# EUROPEAN PATENT SPECIFICATION

(11) **EP 3 344 766 B1**
(45) Date of publication and mention of the grant of the patent: **04.11.2020**
(21) Application number: 16766178.4
(22) Date of filing: 01.09.2016
(51) Int. Cl.: C12N 15/10, C12Q 1/68, C12N 15/117, C12N 15/85

(54) **SYSTEMS AND METHODS FOR SELECTION OF GRNA TARGETING STRANDS FOR CAS9 LOCALIZATION**
SYSTEME UND VERFAHREN ZUR SELEKTION VON GRNS TARGETING STRÄNGEN ZUR CAS9 LOKALISATION
SYSTÈMES ET PROCÉDÉS DE SÉLECTION DE BRINS CIBLANT L'ARNG POUR LA LOCALISATION DE CAS9

(30) Priority: 01.09.2015 US 201562212870 P
(43) Date of publication of application: 11.07.2018
(73) Proprietor: Dana Farber Cancer Institute, Inc., Boston, Massachusetts 02215 (US)
(72) Inventor: NOVINA, Carl, Newton, Massachusetts 02459 (US); MEISTER, Glenna, Boston, Massachusetts 02215 (US)
(74) Representative: Haseltine Lake Kempner LLP
(86) International application number: PCT/US2016/049921
(87) International publication number: WO 2017/040793

(56) References cited:
- WO-A1-2015/021426
- WO-A2-2015/089486
- WO-A2-2016/103233
- LUKE A. GILBERT ET AL: "CRISPR-Mediated Modular RNA-Guided Regulation of Transcription in Eukaryotes", CELL, vol. 154, no. 2, 1 July 2013 (2013-07-01), pages 442-451, XP055321615, US ISSN: 0092-8674, DOI: 10.1016/j.cell.2013.06.044
- MEISTER G E ET AL: "An engineered split M.HhaI-zinc finger fusion lacks the intended methyltransferase specificity", BIOCHEMICAL AND BIOPHYSICAL RESEARCH COMMUNICATIONS, ELSEVIER, AMSTERDAM, NL, vol. 377, no. 1, 5 December 2008 (2008-12-05), pages 226-230, XP025587455, ISSN: 0006-291X, DOI: 10.1016/J.BBRC.2008.09.099 [retrieved on 2008-10-01]
- LUKE A. GILBERT ET AL: "CRISPR-Mediated Modular RNA-Guided Regulation of Transcription in Eukaryotes", CELL, vol. 154, no. 2, 1 July 2013 (2013-07-01), pages 442-S5, XP055167546, ISSN: 0092-8674, DOI: 10.1016/j.cell.2013.06.044
- LEI S. QI ET AL: "Repurposing CRISPR as an RNA-Guided Platform for Sequence-Specific Control of Gene Expression", CELL, vol. 152, no. 5, 1 February 2013 (2013-02-01), pages 1173-1183, XP055299671, US ISSN: 0092-8674, DOI: 10.1016/j.cell.2013.02.022
- HAN XU ET AL: "Sequence determinants of improved CRISPR sgRNA design", GENOME RESEARCH, vol. 25, no. 8, 1 August 2015 (2015-08-01) , pages 1147-1157, XP055321186, US ISSN: 1088-9051, DOI: 10.1101/gr.191452.115

## Description

### RELATED APPLICATIONS

This application claims priority to, and the benefit of, U.S. Provisional Application No. 62/212,870, filed September 1, 2015.

### FIELD OF THE INVENTION

The present invention relates generally to systems and methods for selection of gRNA targeting strands for epigenetic gene regulation and for identifying functionally repressive CpGs sites in a promoter.

### GOVERNMENT INTEREST

This invention was made with government support under grant number DK105602 awarded by The National Institutes of Health and under grant number 1505793 awarded by The National Science Foundation. The government has certain rights in the invention.

### BACKGROUND OF THE INVENTION

Cas9-CRISPR is an endogenous, RNA-based adaptive immune system for bacteria. The Cas9-CRISPR system can be repurposed as a DNA binding domain which can be leveraged for epigenetic reprogramming by localizing genetic activators or repressors to specific genes. The Cas9 protein binds a guide RNA (gRNA) which directs the complex to DNA sequences that are complementary to the gRNA. Therefore, a single Cas9 fusion protein can be targeted to multiple sites simply by transfecting cells with multiple different gRNAs. In many cases multiple sites will need to be targeted, however exact sites that will yield optimal results are typically unknown. The present invention solves this problem by providing a cell based assay that allows for the rapid identification of specific gRNAs for the activation or repression of new promotors.

### SUMMARY OF THE INVENTION

The present invention provides a mammalian cell expressing an expression cassette, wherein the expression cassette comprises: (a) a first promoter sequence operably linked to a nucleic acid sequence encoding (i) a fusion protein comprising a deactivated Cas9 and an epigenetic modifying enzyme and (ii) a selectable marker; and (b) a second promoter sequence operably linked to a nucleic acid sequence encoding at least one deactivated Cas9 specific trans-activating crRNA (TracrRNA), wherein said cell is transfected with a reporter plasmid comprising a backbone that is free of any methylation sites, the reporter plasmid having a promoter sequence from a gene of interest inserted upstream of a nucleic acid encoding a first fluorescent protein and a control promoter sequences inserted upstream of a nucleic acid encoding a second fluorescent protein.

The present invention also provides a mammalian cell expressing an expression cassette, wherein the expression cassette comprises: (a) a first promoter sequence operably linked to a nucleic acid sequence encoding (i) a first fusion protein comprising a deactivated Cas9 and a first portion of a bifurcated epigenetic modifying enzyme, (ii) and a second fusion protein comprising a deactivated Cas9 and a second portion of a bifurcated epigenetic modifying enzyme, and (iii) a selectable marker; and (b) a second promoter sequence operably linked to a nucleic acid sequence encoding at least one deactivated Cas9-specific trans-activating crRNA (TracrRNA), wherein said cell is transfected with a reporter plasmid comprising a backbone that is free of any methylation sites, the reporter plasmid having a promoter sequence from a gene of interest inserted upstream of a nucleic acid encoding a first fluorescent protein and a control promoter sequences inserted upstream of a nucleic acid encoding a second fluorescent protein.

The present invention further provides a mammalian cell expressing an expression cassette, wherein the expression cassette comprises: (a) a first promoter sequence operably linked to a nucleic acid sequence encoding a first fusion protein comprising a deactivated Cas9 and a first portion of a bifurcated epigenetic modifying enzyme, and a second fusion protein comprising a deactivated Cas9 and a second portion of a bifurcated epigenetic modifying enzyme, wherein said first promoter sequence is a bidirectional inducible promoter; (b) a second promoter sequence operably linked to a selectable marker; and (c) a third promoter sequence operably linked to a nucleic acid sequence encoding at least one deactivated Cas9-specific trans-activating crRNA (TracrRNA), wherein said cell is transfected with a reporter plasmid comprising a backbone that is free of any methylation sites, the reporter plasmid having a promoter sequence from a gene of interest inserted upstream of a nucleic acid encoding a first fluorescent protein and a control promoter sequences inserted upstream of a nucleic acid encoding a second fluorescent protein.

The present invention further provides a system comprising: (a) a mammalian cell of the invention; and (b) a plurality of guide RNAs (gRNAs) specific for the gene of interest.

The present invention further provides a method of determining the functionality of a dCAS9-epigenetic modifying enzyme fusion comprising: (a) contacting a mammalian cell of the invention with a plurality of crRNAs specific for the gene of interest; and (b) detecting fluorescence of the first and second fluorescent protein, if present, wherein (i) the presence of fluorescence of the second fluorescent protein and the absence of fluorescence of the first fluorescent protein indicates that the dCAS9-fusion is functional; or (ii) the presence of fluorescence of the both the first and second indicates that the dCAS9-fusion is functional.

The present invention further provides a method of identifying a functionally repressive CpG site in promoter of a gene of interest comprising: (a) contacting a mammalian cell of the invention - wherein the promoter from the gene of interest is methylation sensitive and the control promoter sequence is methylation insensitive and wherein the epigenetic modifying enzyme is a methyltransferase - with a plurality of crRNAs specific for the gene of interest; (b) detecting fluorescence of the first and second fluorescent protein, if present; (c) identifying a cell expressing the second fluorescent protein and not the first fluorescent protein; and (d) performing bisulfite sequencing analysis on the cell of step (c) to identifying the functionally repressive CpG site.

The present invention further provides a method of identifying a functionally repressive CpG site in promoter of a gene of interest comprising: (a) methylating the promoters in the reporter plasmid which is transfected into a mammalian cell of the invention, wherein the promoter from the gene of interest is methylation sensitive and the control promoter sequence is methylation insensitive and wherein the epigenetic modifying enzyme is a demethylase; (b) contacting the cell of step (a) with a plurality of crRNAs specific for the gene of interest; (c) detecting fluorescence of the first and second fluorescent protein, if present; (d) identifying a cell expressing both the first fluorescent protein and the second fluorescent protein; and (e) performing bisulfite sequencing analysis on the cell of step (d) to identifying the functionally repressive CpG site.

The present invention further provides a method of identifying a crRNA that specifically targets a promoter of a gene of interest comprising: (a) contacting a mammalian cell of the invention - wherein the promoter from the gene of interest and the control promoter sequence are methylation sensitive and wherein the epigenetic modifying enzyme is a methyltransferase - with a plurality of crRNAs specific for the gene of interest; (b) detecting fluorescence of the first and second fluorescent protein, if present; and (c) identifying a cell expressing the second fluorescent protein and not the first fluorescent protein.

The present invention further provides a method of identifying a crRNA that specifically targets a promoter of a gene of interest comprising: (a) methylating the promoters in the reporter plasmid which is transfected into the mammalian cell of the invention, wherein the promoter from the gene of interest and the control promoter sequence are methylation sensitive and wherein the epigenetic modifying enzyme is a demethylase; (b) contacting the cell of step (a) with a plurality of crRNAs specific for the gene of interest; (c) detecting fluorescence of the first and second fluorescent protein, if present; and (d) identifying a cell expressing the second fluorescent protein and the not the first fluorescent protein.

Further aspects and embodiments of invention are recited in the appended claims.

Further disclosed is an expression cassette containing a first promoter sequence operably linked to a nucleic acid sequence encoding (i) a fusion protein comprising a deactivated Cas9 and an epigenetic modifying enzyme and (ii) a selectable marker; and a second promoter sequence operably linked to a nucleic acid sequence encoding at least one deactivated Cas9 specific trans-activating crRNA (TracrRNA). Optionally, the expression cassette contains a translation initiation sequence or self-cleaving peptide sequence located between the fusion protein and the selectable marker.

In a further aspect the disclosure relates to an expression cassette containing a first promoter sequence operably linked to a nucleic acid sequence encoding (i) a first fusion protein comprising a deactivated Cas9 and a first portion of a bifurcated epigenetic modifying *enzyme,* (ii) and a second fusion protein comprising a deactivated Cas9 and a second portion of a bifurcated epigenetic modifying enzyme, and (iii) a selectable marker; and a second promoter sequence operably linked to a nucleic acid sequence encoding at least one deactivated Cas9-specific trans-activating crRNA TracrRNA). Optionally, the expression cassette further contains a first translation initiation sequence or a self-cleaving peptide sequence located between the first fusion protein and the second fusion protein and a second translation initiation sequence or self-cleaving peptide sequence located between the second fusion protein and the selectable marker.

The translation initiation sequence is an internal ribosome entry site (IRES), sequence. The self-cleaving peptide sequence is a T2A sequence or an E2A sequence.

In yet another aspect the disclosure relates to an expression cassette containing a first promoter sequence operably linked to a nucleic acid sequence encoding a first fusion protein comprising a deactivated Cas9 and a first portion of a bifurcated epigenetic modifying enzyme, and a second fusion protein comprising a deactivated Cas9 and a second portion of a bifurcated epigenetic modifying enzyme wherein said first promoter is a bidirectional inducible promoter, a second promoter sequence operably linked to a selectable marker; and a third promoter sequence operably linked to a nucleic acid sequence encoding at least one deactivated Cas9-specific trans-activating crRNA (TracrRNA).

The epigenetic modifying enzyme is a methyltransferase, a demethylase, or VP64.

The promoter is a constitutive promoter or an inducible promoter.

The present disclosure provides a mammalian cell expressing the expression cassette described herein. Said mammalian cell is transfected with a reporter plasmid comprising a backbone that is free of any methylation sites, said reporter plasmid having a promoter sequence from a gene of interest inserted upstream of a nucleic acid encoding a first fluorescent protein and a control promoter sequences inserted upstream of a nucleic acid encoding a second fluorescent protein. The promoter from the gene of interest is methylation sensitive and the control promoter sequence is methylation insensitive. Alternatively, the promoter from the gene of interest and the control promoter sequence are methylation sensitive.

The epigenetic modifying enzyme is a methyltransferase or a demethylase.

In some aspects the mammalian cell of the invention is transfected with a plurality of crRNAs specific for the gene of interest.

The invention further provides a system comprising the mammalian cell of the invention a reporter plasmid comprising a backbone that is free of any methylation sites, said reporter plasmid having a promoter sequence from a gene of interest inserted upstream of a nucleic acid encoding a first fluorescent protein and a control promoter sequences inserted upstream of a nucleic acid encoding a second fluorescent protein; and a plurality of guide RNAs (gRNAs) specific for the gene of interest.

The invention also provides methods of determining the functionality of a dCAS9-epigenetic modifying enzyme fusion by contacting the mammalian cell having a reporter plasmid with a plurality of crRNAs specific for the gene of interest; and detecting fluorescence of the first and second fluorescent protein if present. The presence of fluorescence of the second fluorescent protein and the absence of fluorescence of the first fluorescent protein indicates that the dCAS9-fusion is functional. Whereas, the presence of fluorescence of the both the first and second indicates that the dCAS9-fusion is functional.

In another aspect the invention provides methods of identifying a functionally repressive CpG site in promoter of a gene of interest comprising contacting a mammalian cell with a plurality of crRNAs specific for the gene of interest; detecting fluorescence of the first and second fluorescent protein if present. When the epigenetic modifying enzyme is a methyltransferase and the reporter plasmid has a promoter from the gene of interest that methylation sensitive, and a control promoter sequence is methylation insensitive identifying a cell expressing the second fluorescent protein and not the first fluorescent protein and performing bisulfite sequencing analysis on the identified cell to identifying the functionally repressive CpG site.

The invention also provides methods of identifying a functionally repressive CpG site in promoter of a gene of interest comprising methylating the promoters in the reporter plasmid which is transfected into the mammalian cell and contacting the cell with a plurality of crRNAs specific for the gene of interest. When the epigenetic modifying enzyme is a demethylase and the reporter plasmid has a promoter from the gene of interest that methylation sensitive and a control promoter sequence is methylation insensitive detecting fluorescence of the first and second fluorescent protein if present; identifying a cell expressing both the first fluorescent protein and the second fluorescent protein and performing bisulfite sequencing analysis on the identified cell to identifying the functionally repressive CpG site.

In another aspect the invention provides methods of identifying a crRNA that specifically targets a promoter of a gene of interest by contacting a mammalian cell according to the invention with a plurality of crRNAs specific for the gene of interest; detecting fluorescence of the first and second fluorescent protein if present. When the epigenetic modifying enzyme is a methyltransferase and the reporter plasmid has a promoter from the gene of interest and the control promoter is methylation sensitive identifying a cell expressing the second fluorescent protein and not the first fluorescent protein.

In a further aspect the invention provides methods of identifying a crRNA that specifically targets a promoter of a gene of interest by methylating the promoters in the reporter plasmid which is transfected into a mammalian cell according to the invention, contacting the cell with a plurality of crRNAs specific for the gene of interest; detecting fluorescence of the first and second fluorescent protein if present. When the epigenetic modifying enzyme is a demethylase and the reporter plasmid has a promoter from the gene of interest and the control promoter is methylation sensitive identifying a cell expressing the second fluorescent protein and not the first fluorescent protein.

Unless otherwise defined, all technical and scientific terms used herein have the same meaning as commonly understood by one of ordinary skill in the art to which this invention pertains. Although methods and materials similar or equivalent to those described herein can be used in the practice of the present invention, suitable methods and materials are described below.

In cases of conflict, the present specification, including definitions, will control. In addition, the materials, methods, and examples described herein are illustrative only and are not intended to be limiting.

Other features and advantages of the invention will be apparent from and encompassed by the following detailed description and claims.

### BRIEF DESCRIPTION OF THE DRAWINGS

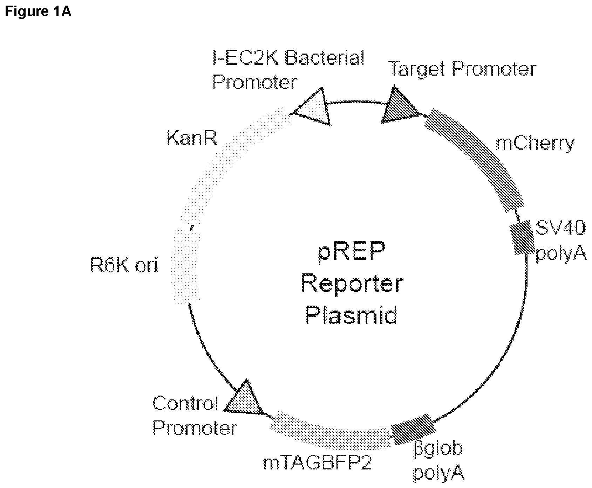

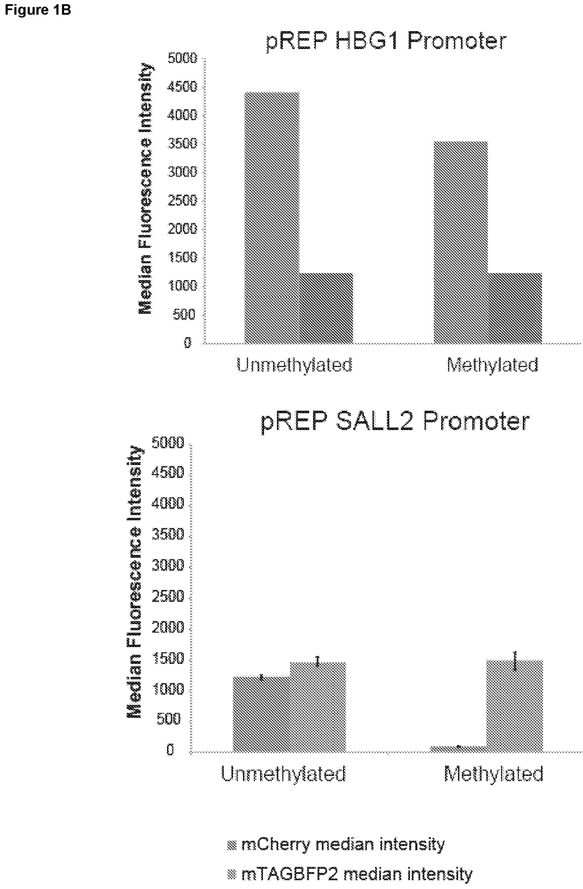

**Figure 1****:** Schematic of pREP reporter plasmid **(****Figure 1A****).** Two promoters were tested for expression of unmethylated and methylated promoters. HBG1 contains only seven CpG sites and does not contain a CpG island. However, methylation of the promoter decreases mCherry median fluorescence ∼20% while mTAGBFP2 median fluorescence is constant. Methylation of the SALL2 promoter, which does contain a classic CpG island, shows a decrease of over 90% of mCherry fluorescence upon methylation **(****Figure 1B****)**.

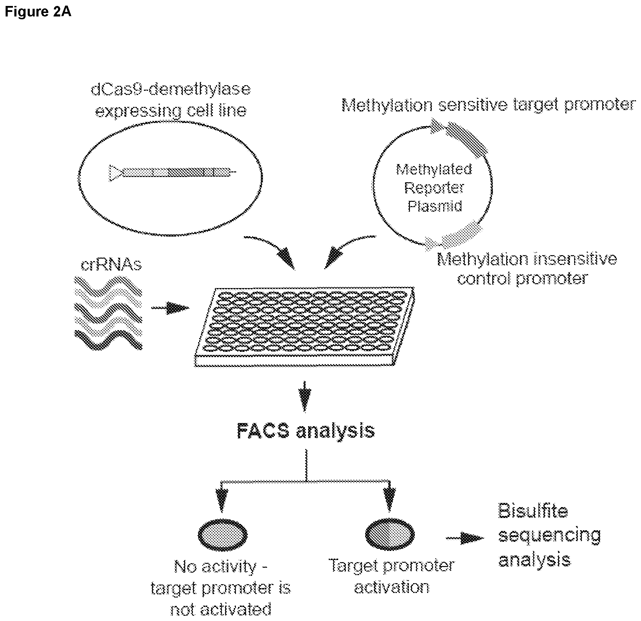

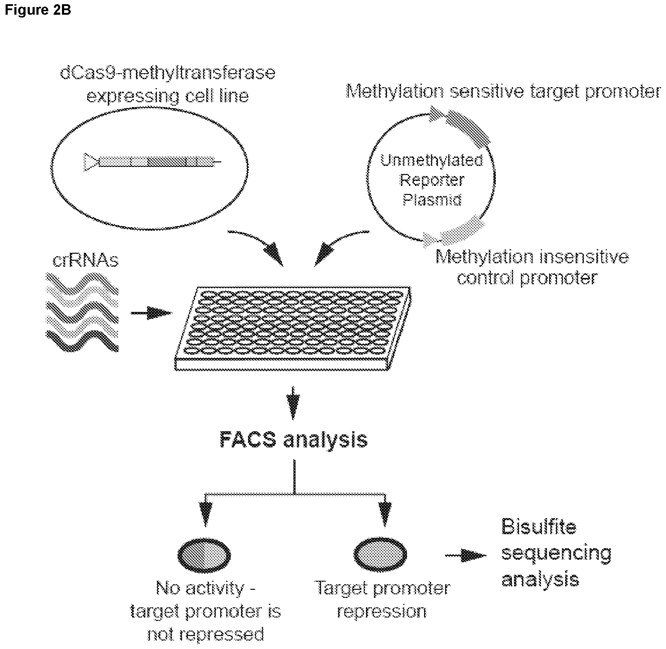

**Figure 2****:** Assay for identification of functionally repressive CpGs and site-specific guide RNAs using an epigenetic modifying enzyme (EME)-dCas9 fusion system. Schematic of a reporter assay designed for evaluating site-specific (**Figure 2A**) demethylation or (**Figure 2B**) methylation by specific crRNAs. Reverse transfection of stable dCas9-EME cells with a reporter plasmid and crRNA libraries will be carried out in 96-well format followed by FACS analysis and bisulfite sequencing analysis will be performed on cells showing target activity of either the demethylase (activation) or methyltransferase (repression).

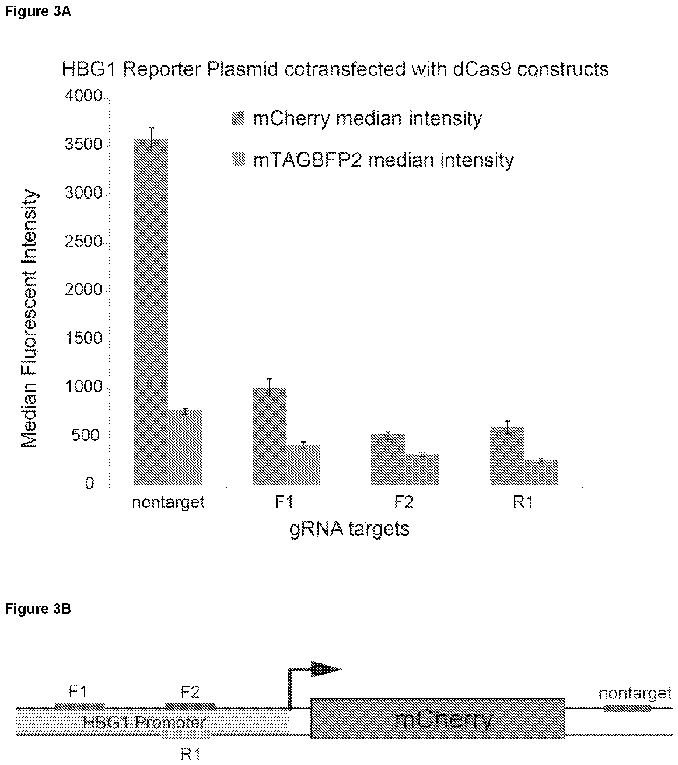

**Figure 3**. Targeting of deactivated Cas9 (dCas9) constructs for repression of the HBG1 promoter. dCas9 was expressed in cells lines with our unmethylated dual-fluorescent reporter and single guide strands (sgRNA). Median fluorescent intensity was determined for different dCas9 gRNA targets **(****Figure 3A****)** and mCherry expression was significantly reduced with sgRNA's targeting the HBG1 promoter vs a non-target site downstream of the mCherry gene **(****Figure 3B****)**.

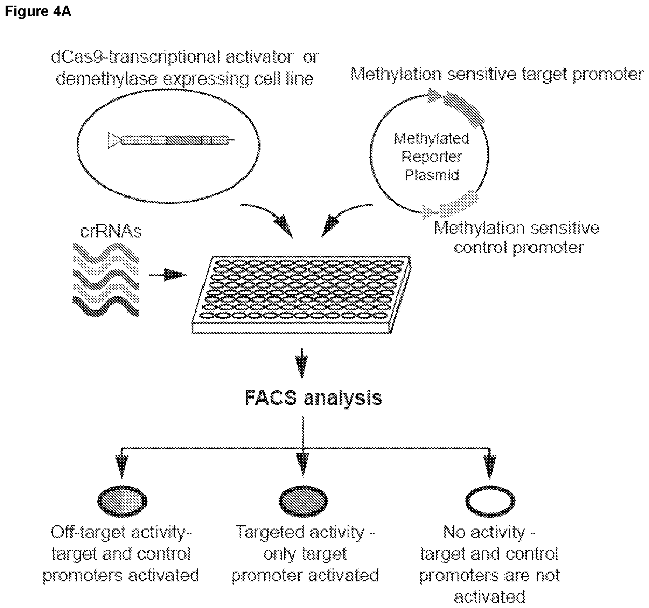

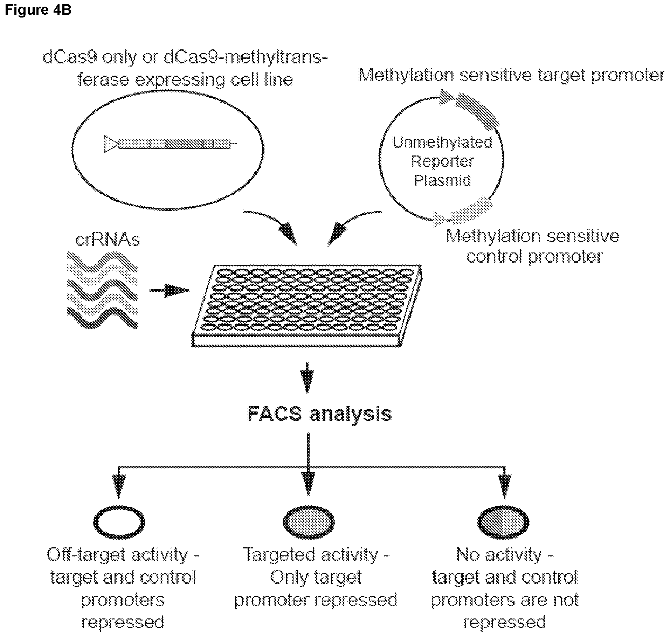

**Figure 4****.** Assay for evaluating off target effects of crRNA libraries using an epigenetic modifying enzyme (EME)-dCas9 fusion system. Schematic of a reporter assay designed for evaluating off target effects of a **(****Figure 4A****)** dCas9-demethylase or **(****Figure 4B****)** dCas9-methyltransferase using specific crRNA libraries. Reverse transfection of stable dCas9-EME cells will be carried out in 96-well format and cells will be evaluated by FACS to assess if there is no activity, targeted activity or off-target activity for each unique crRNA combination.

### DETAILED DESCRIPTION OF THE INVENTION

The present invention provides systems and methods that allow for screening multiple Cas9 guide RNA localization points in a gene promoter region. This cell-based activity assay allows users to: (1) confirm the activity of functional Cas9 fusions, (2) rapidly assess on-target and off-target modifications of promoters, and (3) identify efficient and specific gRNAs for the activation or repression of new promoters.

The systems and methods of the invention are based in part upon an dual fluorescent reporter vector (See, *e.g.*, WO 2016/103233)
and a stable cell expressing a deactivated Cas9s (dCas9) fused to epigenetic modifying enzymes (EMEs) using a mammalian promoter (*e.g.,* inducible or constitutive) along with a tracrRNA specific to the dCas9's used. Cotransfecting the stable cells expressing deactivated Cas9s (dCas9) fused to epigenetic modifying enzymes (EMEs) with short crRNA libraries specific to the gene of interest along with the corresponding dual fluorescent reporter plasmid allows for the rapid selection of efficient control of the target promoter but not the control promoter. This assay can be used to select for a variety of Cas9 fusion partners including transcriptional activators, DNA methyltransferases, and DNA demethylases as well as dCas9 alone (CRISPR).

The invention provides a user-friendly reporter plasmid and library of different stable cell lines expressing deactivated Cas9 (dCas9) and epigenetic modifying enzyme (EME) fusions for rapidly screening gRNAs and for identifying repressive methylation sites in mammalian promoters. This can either be done by methylating sites (dCas9-methyltransferase fusions) to silence genes or de-methylating sites (dCas9-demethylating enzyme) to activate genes.

### Reporter Plasmid

The reporter plasmid is a CpG-free backbone engineered with multiple cloning sites for rapid and directional insertion of test promoter fragments (*i.e.*, a promoter sequence from a gene of interest) upstream of a first fluorescent protein (*e.g.,* red fluorescent protein (mCherry). A methylation-resistant control promoter is cloned upstream of a second fluorescent protein (*e.g*., blue fluorescent protein (BFP)) to allow for normalization of the first fluorescent protein expression **(****Figure 1A**). By utilizing a reporter plasmid we ensure that (1) the promoter is 100% unmethylated or methylated initially, (2) the promoter is not blocked by higher chromatin structures and is accessible to our dCas9-EME fusions, and (3) gene expression is easily quantifiable by flow cytometry analysis.

### Deactivated Cas9 (dCas9)-Epigenetic Modifying Enzyme Fusion Protein Expression Cassettes

The invention provides expression cassettes encoding fusion proteins containing a deactivated Cas9 and an epigenetic modifying enzyme such as a methyltransferase, a demethylase or a VP64 transcriptional activator. In some aspects the epigenetic modifying enzyme is split into two domains. In addition to the nucleic acid encoding the fusion protein, the cassette further includes a promoter, a selectable marker and a TracrRNA under the control of a U6 or HI promoter. Optionally, the cassette further includes one or more translation initiation sequences such as an internal ribosome entry site (IRES) or a self-cleaving peptide (T2A or E2A).

The promoter is a constitutive promoter or an inducible promoter. Optionally, the promoter is bidirectional. Constitutive promoters include for example PGK1, EF1alpha, CMV, SFFV, Ubc, SV40, and the CAG promoters. Many inducible promoters systems suitable for use in mammalian cells are known on the art. For example, the tetracycline-inducible system (Tet-On), the cumate inducible system, the ecdysone inducible system, the pristinamycin inducible system (Pip-ON), or the erythromycin inducible system (Eₒₙ/E.REX system).

Dependent upon the inducible promoter system utilized it may be necessary to include a reverse transactivator or transrepressor sequence to control the inducible promoter.

The selectable marker is for example, a fluorescent protein or an antibiotic selection marker.

### Cells

The invention also relates to a cell containing the expression cassettes according to the invention. This may be a cell from any species. In particular, prokaryotic and eukaryotic cells that contain the expression cassettes according to the invention are encompassed by the invention. Preferably, the cell is a vertebrate cell, more preferably a human cell. In some embodiments, the cell is a HEK293, HEK293T, K562, HELA or other immortalized cell line.

Cells containing expression cassettes according to the invention may be prepared by conventional transfection methods known in the art. For example, chemical transfection using calcium phosphate can be used (see F L Graham et al., Virology 1973, 52(2): 456-467), or using dendrimers (Colander H L Fu et al., Journal of Control Release 2007, 124(3):181-188) or using cationic polymers (see EP 1505089). Further methods include lipofection (see Felgner P L et al., PNAS, 1987, 84(21): 7413-7417) electroporation (see E. Neumann, et al, EMBO J. 1982, 1(7):841-845), optical transfection (see M. Tsukakoshi et al., Applied Physics B-Photophysics and Laser Chemistry 1984, 35(3): 135-140), magnetofection (see F. Scherer et al., Gene Ther., 2009, 9(2): 102-109) or impalefection (see T E McKnight et al., Nano Letters 2004, 4(7): 1213-1219). Particle-based techniques such as gene gun can be also used (see U.S. Pat. No. 5,219,746). Preferred methods are calcium phosphate transfection, lipofection and electroporation.

### Definitions

Before describing the invention in detail, it is to be understood that this invention is not limited to particular biological systems or cell types. It is also to be understood that the terminology used herein is for the purpose of describing particular embodiments only, and is not intended to be limiting. As used in this specification and the appended claims, the singular forms "a", "an" and "the" include plural referents unless the content clearly dictates otherwise. Thus, for example, reference to "a cell" includes combinations of two or more cells, or entire cultures of cells; reference to "a polynucleotide" includes, as a practical matter, many copies of that polynucleotide. Unless defined herein and below in the reminder of the specification, all technical and scientific terms used herein have the same meaning as commonly understood by one of ordinary skill in the art to which the invention pertains.

As used herein, a "trans-activating crRNA" (tracrRNA) is a small trans-encoded RNA. tracrRNA is also known as a Cas9 binding hairpin or Cas9 handle, means a hairpin structure, which can bind to an RNA-guided nuclease such as Cas9A tracrRNA links crRNA to the RNA guided nuclease.

As used herein, "crRNA"means a short RNA *(e.g.,* 15-30 or 17-25 nucleotides) complementary to a segment of a strand of DNA molecule being targeted for cleavage by an RNA guided nuclease.

Together, the tracrRNA and the crRNA anneal to form the guide RNA (gRNA) The guide RNA directs the RNA guided nuclease to cut DNA within the segment forming the duplex region or complementary segment of the other strand

A fusion of a tracrRNA and a crRNA is referred to as a chimeric RNA or sgRNA. The tracrRNA and crRNA can be fused directly or via a short RNA linker *(e.g.,* 3-10 bases).

As used herein, "DNA binding protein portion" is a segment of a DNA binding protein or polypeptide capable of specifically binding to a particular DNA sequence. The binding is specific to a particular DNA sequence site. The DNA binding protein portion may include a truncated segment of a DNA binding protein or a fragment of a DNA binding protein.

As used herein, "binds sufficiently close" means the contacting of a DNA molecule by a protein at a position on the DNA molecule near enough to a predetermined methylation site on the DNA molecule to allow proper functioning of the protein and allow specific methylation of the predetermined methylation site.

As used herein, "a promoter sequence of a target gene" or "a promoter sequence of a gene of interest" is at least a portion of a non-coding DNA sequence which directs the expression of the target gene. The portion of the non-coding DNA sequence may be in the 5'-prime direction or in the 3'-prime direction from the coding region of the target gene. The portion of the non-coding DNA sequence may be located in an intron of the target gene.

The promoter sequence of the target gene or gene of interest may be a 5' long terminal repeat sequence of a human immunodeficiency virus-1 proviral DNA.

As used herein "specifically methylate" means to bond a methyl group to a methylation site in a DNA sequence, which methylation site may be -CpG-, wherein the methylation is restricted to particular methylation site(s) and the methylation is not random.

As used herein "specifically demethylate" means to remove a methyl group from a methylation site in a DNA sequence, which methylation site may be -CpG-, wherein the demethylation is restricted to particular methylation site(s) and the demethylation is not random.

As used herein, the terms "polynucleotide," "nucleic acid," "oligonucleotide," "oligomer," "oligo" or equivalent terms, refer to molecules that comprises a polymeric arrangement of nucleotide base monomers, where the sequence of monomers defines the polynucleotide. Polynucleotides can include polymers of deoxyribonucleotides to produce deoxyribonucleic acid (DNA), and polymers of ribonucleotides to produce ribonucleic acid (RNA). A polynucleotide can be single-stranded or double-stranded. When single stranded, the [polynucleotide can correspond to the sense or antisense strand of a gene. A single-stranded polynucleotide can hybridize with a complementary portion of a target polynucleotide to form a duplex, which can be a homoduplex or a heteroduplex.

The length of a polynucleotide is not limited in any respect. Linkages between nucleotides can be internucleotide-type phosphodiester linkages, or any other type of linkage. A polynucleotide can be produced by biological means (*e.g.,* enzymatically), either in vivo (in a cell) or in vitro (in a cell-free system). A polynucleotide can be chemically synthesized using enzyme-free systems. A polynucleotide can be enzymatically extendable or enzymatically non-extendable.

By convention, polynucleotides that are formed by 3'-5' phosphodiester linkages (including naturally occurring polynucleotides) are said to have 5'-ends and 3'-ends because the nucleotide monomers that are incorporated into the polymer are joined in such a manner that the 5' phosphate of one mononucleotide pentose ring is attached to the 3' oxygen (hydroxyl) of its neighbor in one direction via the phosphodiester linkage. Thus, the 5'-end of a polynucleotide molecule generally has a free phosphate group at the 5' position of the pentose ring of the nucleotide, while the 3' end of the polynucleotide molecule has a free hydroxyl group at the 3' position of the pentose ring. Within a polynucleotide molecule, a position that is oriented 5' relative to another position is said to be located "upstream," while a position that is 3' to another position is said to be "downstream." This terminology reflects the fact that polymerases proceed and extend a polynucleotide chain in a 5' to 3' fashion along the template strand. Unless denoted otherwise, whenever a polynucleotide sequence is represented, it will be understood that the nucleotides are in 5' to 3' orientation from left to right.

As used herein, it is not intended that the term "polynucleotide" be limited to naturally occurring polynucleotide structures, naturally occurring nucleotides sequences, naturally occurring backbones or naturally occurring internucleotide linkages. One familiar with the art knows well the wide variety of polynucleotide analogues, unnatural nucleotides, non-natural phosphodiester bond linkages and internucleotide analogs that find use with the invention.

As used herein, the expressions "nucleotide sequence," "sequence of a polynucleotide," "nucleic acid sequence," "polynucleotide sequence", and equivalent or similar phrases refer to the order of nucleotide monomers in the nucleotide polymer. By convention, a nucleotide sequence is typically written in the 5' to 3' direction. Unless otherwise indicated, a particular polynucleotide sequence of the invention optionally encompasses complementary sequences, in addition to the sequence explicitly indicated.

As used herein, the term "gene" generally refers to a combination of polynucleotide elements, that when operatively linked in either a native or recombinant manner, provide some product or function. The term "gene" is to be interpreted broadly, and can encompass mRNA, cDNA, cRNA and genomic DNA forms of a gene. In some uses, the term "gene" encompasses the transcribed sequences, including 5' and 3' untranslated regions (5'-UTR and 3'-UTR), exons and introns. In some genes, the transcribed region will contain "open reading frames" that encode polypeptides. In some uses of the term, a "gene" comprises only the coding sequences (*e.g.,* an "open reading frame" or "coding region") necessary for encoding a polypeptide. In some aspects, genes do not encode a polypeptide, for example, ribosomal RNA genes (rRNA) and transfer RNA (tRNA) genes. In some aspects, the term "gene" includes not only the transcribed sequences, but in addition, also includes non-transcribed regions including upstream and downstream regulatory regions, enhancers and promoters. The term "gene" encompasses mRNA, cDNA and genomic forms of a gene.

In some aspects, the genomic form or genomic clone of a gene includes the sequences of the transcribed mRNA, as well as other non-transcribed sequences which lie outside of the transcript. The regulatory regions which lie outside the mRNA transcription unit are termed 5' or 3' flanking sequences. A functional genomic form of a gene typically contains regulatory elements necessary, and sometimes sufficient, for the regulation of transcription. The term "promoter" is generally used to describe a DNA region, typically but not exclusively 5' of the site of transcription initiation, sufficient to confer accurate transcription initiation. In some aspects, a "promoter" also includes other cis-acting regulatory elements that are necessary for strong or elevated levels of transcription, or confer inducible transcription. In some embodiments, a promoter is constitutively active, while in alternative embodiments, the promoter is conditionally active (*e.g.,* where transcription is initiated only under certain physiological conditions).

Generally, the term "regulatory element" refers to any cis-acting genetic element that controls some aspect of the expression of nucleic acid sequences. In some uses, the term "promoter" comprises essentially the minimal sequences required to initiate transcription. In some uses, the term "promoter" includes the sequences to start transcription, and in addition, also include sequences that can upregulate or downregulate transcription, commonly termed "enhancer elements" and "repressor elements," respectively.

Specific DNA regulatory elements, including promoters and enhancers, generally only function within a class of organisms. For example, regulatory elements from the bacterial genome generally do not function in eukaryotic organisms. However, regulatory elements from more closely related organisms frequently show cross functionality. For example, DNA regulatory elements from a particular mammalian organism, such as human, will most often function in other mammalian species, such as mouse. Furthermore, in designing recombinant genes that will function across many species, there are consensus sequences for many types of regulatory elements that are known to function across species, *e.g.,* in all mammalian cells, including mouse host cells and human host cells.

As used herein, the expressions "in operable combination," "in operable order," "operatively linked," "operatively joined" and similar phrases, when used in reference to nucleic acids, refer to the operational linkage of nucleic acid sequences placed in functional relationships with each other. For example, an operatively linked promoter, enhancer elements, open reading frame, 5' and 3' UTR, and terminator sequences result in the accurate production of an RNA molecule. In some aspects, operatively linked nucleic acid elements result in the transcription of an open reading frame and ultimately the production of a polypeptide (*i.e.,* expression of the open reading frame).

As used herein, the term "genome" refers to the total genetic information or hereditary material possessed by an organism (including viruses), *i.e.,* the entire genetic complement of an organism or virus. The genome generally refers to all of the genetic material in an organism's chromosome(s), and in addition, extra-chromosomal genetic information that is stably transmitted to daughter cells *(e.g.,* the mitochondrial genome). A genome can comprise RNA or DNA. A genome can be linear (mammals) or circular (bacterial). The genomic material typically resides on discrete units such as the chromosomes.

As used herein, a "polypeptide" is any polymer of amino acids (natural or unnatural, or a combination thereof), of any length, typically but not exclusively joined by covalent peptide bonds. A polypeptide can be from any source, *e.g.,* a naturally occurring polypeptide, a polypeptide produced by recombinant molecular genetic techniques, a polypeptide from a cell, or a polypeptide produced enzymatically in a cell-free system. A polypeptide can also be produced using chemical (non-enzymatic) synthesis methods. A polypeptide is characterized by the amino acid sequence in the polymer. As used herein, the term "protein" is synonymous with polypeptide. The term "peptide" typically refers to a small polypeptide, and typically is smaller than a protein. Unless otherwise stated, it is not intended that a polypeptide be limited by possessing or not possessing any particular biological activity.

As used herein, the expressions "codon utilization" or "codon bias" or "preferred codon utilization" or the like refers, in one aspect, to differences in the frequency of occurrence of any one codon from among the synonymous codons that encode for a single amino acid in protein-coding DNA (where many amino acids have the capacity to be encoded by more than one codon). In another aspect, "codon use bias" can also refer to differences between two species in the codon biases that each species shows. Different organisms often show different codon biases, where preferences for which codons from among the synonymous codons are favored in that organism's coding sequences.

As used herein, the terms "vector," "vehicle," "construct" and "plasmid" are used in reference to any recombinant polynucleotide molecule that can be propagated and used to transfer nucleic acid segment(s) from one organism to another. Vectors generally comprise parts which mediate vector propagation and manipulation (*e.g.,* one or more origin of replication, genes imparting drug or antibiotic resistance, a multiple cloning site, operably linked promoter/enhancer elements which enable the expression of a cloned gene, etc.). Vectors are generally recombinant nucleic acid molecules, often derived from bacteriophages, or plant or animal viruses. Plasmids and cosmids refer to two such recombinant vectors. A "cloning vector" or "shuttle vector" or "subcloning vector" contains operably linked parts that facilitate subcloning steps *(e.g.,* a multiple cloning site containing multiple restriction endonuclease target sequences). A nucleic acid vector can be a linear molecule, or in circular form, depending on type of vector or type of application. Some circular nucleic acid vectors can be intentionally linearized prior to delivery into a cell.

As used herein, the term "expression vector" refers to a recombinant vector comprising operably linked polynucleotide elements that facilitate and optimize expression of a desired gene *(e.g.,* a gene that encodes a protein) in a particular host organism *(e.g.,* a bacterial expression vector or mammalian expression vector). Polynucleotide sequences that facilitate gene expression can include, for example, promoters, enhancers, transcription termination sequences, and ribosome binding sites.

As used herein, the term "host cell" refers to any cell that contains a heterologous nucleic acid. The heterologous nucleic acid can be a vector, such as a shuttle vector or an expression vector. In some aspects, the host cell is able to drive the expression of genes that are encoded on the vector. In some aspects, the host cell supports the replication and propagation of the vector. Host cells can be bacterial cells such as E. coli, or mammalian cells (*e.g.,* human cells or mouse cells). When a suitable host cell (such as a suitable mouse cell) is used to create a stably integrated cell line, that cell line can be used to create a complete transgenic organism.

Methods (*i.e.,* means) for delivering vectors/constructs or other nucleic acids (such as in vitro transcribed RNA) into host cells such as bacterial cells and mammalian cells are well known to one of ordinary skill in the art, and are not provided in detail herein. Any method for nucleic acid delivery into a host cell finds use with the invention.

For example, methods for delivering vectors or other nucleic acid molecules into bacterial cells (termed transformation) such as Escherichia coli are routine, and include electroporation methods and transformation of E. coli cells that have been rendered competent by previous treatment with divalent cations such as CaCl₂.

Methods for delivering vectors or other nucleic acid (such as RNA) into mammalian cells in culture (termed transfection) are routine, and a number of transfection methods find use with the invention. These include but are not limited to calcium phosphate precipitation, electroporation, lipid-based methods (liposomes or lipoplexes) such as Transfectamine® (Life Technologies™) and TransFectin™. (Bio-Rad Laboratories®), cationic polymer transfections, for example using DEAE-dextran, direct nucleic acid injection, biolistic particle injection, and viral transduction using engineered viral carriers (termed transduction, using *e.g.,* engineered herpes simplex virus, adenovirus, adeno-associated virus, vaccinia virus, Sindbis virus), and sonoporation. Any of these methods find use with the invention.

As used herein, the term "recombinant" in reference to a nucleic acid or polypeptide indicates that the material (*e.g.,* a recombinant nucleic acid, gene, polynucleotide, polypeptide, etc.) has been altered by human intervention. Generally, the arrangement of parts of a recombinant molecule is not a native configuration, or the primary sequence of the recombinant polynucleotide or polypeptide has in some way been manipulated. A naturally occurring nucleotide sequence becomes a recombinant polynucleotide if it is removed from the native location from which it originated (*e.g.,* a chromosome), or if it is transcribed from a recombinant DNA construct. A gene open reading frame is a recombinant molecule if that nucleotide sequence has been removed from it natural context and cloned into any type of nucleic acid vector (even if that ORF has the same nucleotide sequence as the naturally occurring gene). Protocols and reagents to produce recombinant molecules, especially recombinant nucleic acids, are well known to one of ordinary skill in the art. In some embodiments, the term "recombinant cell line" refers to any cell line containing a recombinant nucleic acid, that is to say, a nucleic acid that is not native to that host cell.

As used herein, the terms "heterologous" or "exogenous" as applied to polynucleotides or polypeptides refers to molecules that have been rearranged or artificially supplied to a biological system and are not in a native configuration (*e.g.,* with respect to sequence, genomic position or arrangement of parts) or are not native to that particular biological system. These terms indicate that the relevant material originated from a source other than the naturally occurring source, or refers to molecules having a non-natural configuration, genetic location or arrangement of parts. The terms "exogenous" and "heterologous" are sometimes used interchangeably with "recombinant."

As used herein, the terms "native" or "endogenous" refer to molecules that are found in a naturally occurring biological system, cell, tissue, species or chromosome under study. A "native" or "endogenous" gene is a generally a gene that does not include nucleotide sequences other than nucleotide sequences with which it is normally associated in nature (*e.g.,* a nuclear chromosome, mitochondrial chromosome or chloroplast chromosome). An endogenous gene, transcript or polypeptide is encoded by its natural locus, and is not artificially supplied to the cell.

As used herein, the term "marker" most generally refers to a biological feature or trait that, when present in a cell *(e.g.,* is expressed), results in an attribute or phenotype that visualizes or identifies the cell as containing that marker. A variety of marker types are commonly used, and can be for example, visual markers such as color development, *e.g.,* lacZ complementation (.beta.-galactosidase) or fluorescence, *e.g.,* such as expression of green fluorescent protein (GFP) or GFP fusion proteins, RFP, BFP, selectable markers, phenotypic markers (growth rate, cell morphology, colony color or colony morphology, temperature sensitivity), auxotrophic markers (growth requirements), antibiotic sensitivities and resistances, molecular markers such as biomolecules that are distinguishable by antigenic sensitivity (*e.g.,* blood group antigens and histocompatibility markers), cell surface markers (for example H2KK), enzymatic markers, and nucleic acid markers, for example, restriction fragment length polymorphisms (RFLP), single nucleotide polymorphism (SNP) and various other amplifiable genetic polymorphisms.

As used herein, the expressions "selectable marker" or "screening marker" or "positive selection marker" refer to a marker that, when present in a cell, results in an attribute or phenotype that allows selection or segregated of those cells from other cells that do not express the selectable marker trait. A variety of genes are used as selectable markers, *e.g.,* genes encoding drug resistance or auxotrophic rescue are widely known. For example, kanamycin (neomycin) resistance can be used as a trait to select bacteria that have taken up a plasmid carrying a gene encoding for bacterial kanamycin resistance (*e.g.*, the enzyme neomycin phosphotransferase II). Non-transfected cells will eventually die off when the culture is treated with neomycin or similar antibiotic.

A similar mechanism can also be used to select for transfected mammalian cells containing a vector carrying a gene encoding for neomycin resistance (either one of two aminoglycoside phosphotransferase genes; the neo selectable marker). This selection process can be used to establish stably transfected mammalian cell lines. Geneticin (G418) is commonly used to select the mammalian cells that contain stably integrated copies of the transfected genetic material.

As used herein, the expressions "negative selection" or "negative screening marker" refers to a marker that, when present (*e.g.,* expressed, activated, or the like) allows identification of a cell that does not comprise a selected property or trait (*e.g.,* as compared to a cell that does possess the property or trait).

A wide variety of positive and negative selectable markers are known for use in prokaryotes and eukaryotes, and selectable marker tools for plasmid selection in bacteria and mammalian cells are widely available. Bacterial selection systems include, for example but not limited to, ampicillin resistance (.beta.-lactamase), chloramphenicol resistance, kanamycin resistance (aminoglycoside phosphotransferases), and tetracycline resistance. Mammalian selectable marker systems include, for example but not limited to, neomycin/G418 (neomycin phosphotransferase II), methotrexate resistance (dihydropholate reductase; DHFR), hygromycin-B resistance (hygromycin-B phosphotransferase), and blasticidin resistance (blasticidin S deaminase).

As used herein, the term "reporter" refers generally to a moiety, chemical compound or other component that can be used to visualize, quantitate or identify desired components of a system of interest. Reporters are commonly, but not exclusively, genes that encode reporter proteins. For example, a "reporter gene" is a gene that, when expressed in a cell, allows visualization or identification of that cell, or permits quantitation of expression of a recombinant gene. For example, a reporter gene can encode a protein, for example, an enzyme whose activity can be quantitated, for example, chloramphenicol acetyltransferase (CAT) or firefly luciferase protein. Reporters also include fluorescent proteins, for example, green fluorescent protein (GFP) or any of the recombinant variants of GFP, including enhanced GFP (EGFP), blue fluorescent proteins (BFP and derivatives), cyan fluorescent protein (CFP and other derivatives), yellow fluorescent protein (YFP and other derivatives) and red fluorescent protein (RFP and other derivatives).

As used herein, the term "tag" as used in protein tags refers generally to peptide sequences that are genetically fused to other protein open reading frames, thereby producing recombinant fusion proteins. Ideally, the fused tag does not interfere with the native biological activity or function of the larger protein to which it is fused. Protein tags are used for a variety of purposes, for example but not limited to, tags to facilitate purification, detection or visualization of the fusion proteins. Some peptide tags are removable by chemical agents or by enzymatic means, such as by target-specific proteolysis (*e.g.,* by TEV).

Depending on use, the terms "marker," "reporter" and "tag" may overlap in definition, where the same protein or polypeptide can be used as either a marker, a reporter or a tag in different applications. In some scenarios, a polypeptide may simultaneously function as a reporter and/or a tag and/or a marker, all in the same recombinant gene or protein.

As used herein, the term "prokaryote" refers to organisms belonging to the Kingdom Monera (also termed Procarya), generally distinguishable from eukaryotes by their unicellular organization, asexual reproduction by budding or fission, the lack of a membrane-bound nucleus or other membrane-bound organelles, a circular chromosome, the presence of operons, the absence of introns, message capping and poly-A mRNA, a distinguishing ribosomal structure and other biochemical characteristics. Prokaryotes include subkingdoms Eubacteria ("true bacteria") and Archaea (sometimes termed "archaebacteria").

As used herein, the terms "bacteria" or "bacterial" refer to prokaryotic Eubacteria, and are distinguishable from Archaea, based on a number of well-defined morphological and biochemical criteria.

As used herein, the term "eukaryote" refers to organisms (typically multicellular organisms) belonging to the Kingdom Eucarya, generally distinguishable from prokaryotes by the presence of a membrane-bound nucleus and other membrane-bound organelles, linear genetic material (*i.e.,* linear chromosomes), the absence of operons, the presence of introns, message capping and poly-A mRNA, a distinguishing ribosomal structure and other biochemical characteristics.

As used herein, the terms "mammal" or "mammalian" refer to a group of eukaryotic organisms that are endothermic amniotes distinguishable from reptiles and birds by the possession of hair, three middle ear bones, mammary glands in females, a brain neocortex, and most giving birth to live young. The largest group of mammals, the placentals (Eutheria), has a placenta which feeds the offspring during pregnancy. The placentals include the orders Rodentia (including mice and rats) and primates (including humans).

A "subject" in the context of the present invention is preferably a mammal. The mammal can be a human, non-human primate, mouse, rat, dog, cat, horse, or cow, but are not limited to these examples.

As used herein, the term "encode" refers broadly to any process whereby the information in a polymeric macromolecule is used to direct the production of a second molecule that is different from the first. The second molecule may have a chemical structure that is different from the chemical nature of the first molecule.

For example, in some aspects, the term "encode" describes the process of semi-conservative DNA replication, where one strand of a double-stranded DNA molecule is used as a template to encode a newly synthesized complementary sister strand by a DNA-dependent DNA polymerase. In other aspects, a DNA molecule can encode an RNA molecule (*e.g.,* by the process of transcription that uses a DNA-dependent RNA polymerase enzyme). Also, an RNA molecule can encode a polypeptide, as in the process of translation. When used to describe the process of translation, the term "encode" also extends to the triplet codon that encodes an amino acid. In some aspects, an RNA molecule can encode a DNA molecule, *e.g.*, by the process of reverse transcription incorporating an RNA-dependent DNA polymerase. In another aspect, a DNA molecule can encode a polypeptide, where it is understood that "encode" as used in that case incorporates both the processes of transcription and translation.

As used herein, the term "derived from" refers to a process whereby a first component (*e.g.,* a first molecule), or information from that first component, is used to isolate, derive or make a different second component (*e.g.,* a second molecule that is different from the first). For example, the mammalian codon-optimized Cas9 polynucleotides of the invention are derived from the wild type Cas9 protein amino acid sequence. Also, the variant mammalian codon-optimized Cas9 polynucleotides of the invention, including the Cas9 single mutant nickase and Cas9 double mutant null-nuclease, are derived from the polynucleotide encoding the wild type mammalian codon-optimized Cas9 protein.

As used herein, the expression "variant" refers to a first composition (*e.g.,* a first molecule), that is related to a second composition (*e.g.,* a second molecule, also termed a "parent" molecule). The variant molecule can be derived from, isolated from, based on or homologous to the parent molecule. For example, the mutant forms of mammalian codon-optimized Cas9 (hspCas9), including the Cas9 single mutant nickase and the Cas9 double mutant null-nuclease, are variants of the mammalian codon-optimized wild type Cas9 (hspCas9). The term variant can be used to describe either polynucleotides or polypeptides.

As applied to polynucleotides, a variant molecule can have entire nucleotide sequence identity with the original parent molecule, or alternatively, can have less than 100% nucleotide sequence identity with the parent molecule. For example, a variant of a gene nucleotide sequence can be a second nucleotide sequence that is at least 50%, 60%, 70%, 80%, 90%, 95%, 98%, 99% or more identical in nucleotide sequence compare to the original nucleotide sequence. Polynucleotide variants also include polynucleotides comprising the entire parent polynucleotide, and further comprising additional fused nucleotide sequences. Polynucleotide variants also includes polynucleotides that are portions or subsequences of the parent polynucleotide, for example, unique subsequences *(e.g.,* as determined by standard sequence comparison and alignment techniques) of the polynucleotides disclosed herein are also encompassed by the invention.

In another aspect, a polynucleotide variant includes nucleotide sequences that contain minor, trivial or inconsequential changes to the parent nucleotide sequence. For example, minor, trivial or inconsequential changes include changes to nucleotide sequence that (i) do not change the amino acid sequence of the corresponding polypeptide, (ii) occur outside the protein-coding open reading frame of a polynucleotide, (iii) result in deletions or insertions that may impact the corresponding amino acid sequence, but have little or no impact on the biological activity of the polypeptide, (iv) the nucleotide changes result in the substitution of an amino acid with a chemically similar amino acid. In the case where a polynucleotide does not encode for a protein (for example, a tRNA or a crRNA or a tracrRNA), variants of that polynucleotide can include nucleotide changes that do not result in loss of function of the polynucleotide. In another aspect, conservative variants of the disclosed nucleotide sequences that yield functionally identical nucleotide sequences are encompassed by the invention. One of skill will appreciate that many variants of the disclosed nucleotide sequences are encompassed by the invention.

Variant polypeptides are also disclosed. As applied to proteins, a variant polypeptide can have entire amino acid sequence identity with the original parent polypeptide, or alternatively, can have less than 100% amino acid identity with the parent protein. For example, a variant of an amino acid sequence can be a second amino acid sequence that is at least 50%, 60%, 70%, 80%, 90%, 95%, 98%, 99% or more identical in amino acid sequence compared to the original amino acid sequence.

Polypeptide variants include polypeptides comprising the entire parent polypeptide, and further comprising additional fused amino acid sequences. Polypeptide variants also includes polypeptides that are portions or subsequences of the parent polypeptide, for example, unique subsequences (*e.g.,* as determined by standard sequence comparison and alignment techniques) of the polypeptides disclosed herein are also encompassed by the invention.

In another aspect, a polypeptide variant includes polypeptides that contain minor, trivial or inconsequential changes to the parent amino acid sequence. For example, minor, trivial or inconsequential changes include amino acid changes (including substitutions, deletions and insertions) that have little or no impact on the biological activity of the polypeptide, and yield functionally identical polypeptides, including additions of non-functional peptide sequence. In other aspects, the variant polypeptides of the invention change the biological activity of the parent molecule, for example, mutant variants of the Cas9 polypeptide that have modified or lost nuclease activity. One of skill will appreciate that many variants of the disclosed polypeptides are encompassed by the invention.

In some aspects, polynucleotide or polypeptide variants of the invention can include variant molecules that alter, add or delete a small percentage of the nucleotide or amino acid positions, for example, typically less than about 10%, less than about 5%, less than 4%, less than 2% or less than 1%.

As used herein, the term "conservative substitutions" in a nucleotide or amino acid sequence refers to changes in the nucleotide sequence that either (i) do not result in any corresponding change in the amino acid sequence due to the redundancy of the triplet codon code, or (ii) result in a substitution of the original parent amino acid with an amino acid having a chemically similar structure. Conservative substitution tables providing functionally similar amino acids are well known in the art, where one amino acid residue is substituted for another amino acid residue having similar chemical properties (*e.g.,* aromatic side chains or positively charged side chains), and therefore does not substantially change the functional properties of the resulting polypeptide molecule.

The following are groupings of natural amino acids that contain similar chemical properties, where a substitution within a group is a "conservative" amino acid substitution. This grouping indicated below is not rigid, as these natural amino acids can be placed in different grouping when different functional properties are considered. Amino acids having nonpolar and/or aliphatic side chains include: glycine, alanine, valine, leucine, isoleucine and proline. Amino acids having polar, uncharged side chains include: serine, threonine, cysteine, methionine, asparagine and glutamine. Amino acids having aromatic side chains include: phenylalanine, tyrosine and tryptophan. Amino acids having positively charged side chains include: lysine, arginine and histidine. Amino acids having negatively charged side chains include: aspartate and glutamate.

As used herein, the terms "identical" or "percent identity" in the context of two or more nucleic acids or polypeptides refer to two or more sequences or subsequences that are the same ("identical") or have a specified percentage of amino acid residues or nucleotides that are identical ("percent identity") when compared and aligned for maximum correspondence with a second molecule, as measured using a sequence comparison algorithm (*e.g*., by a BLAST alignment, or any other algorithm known to persons of skill), or alternatively, by visual inspection.

The phrase "substantially identical," in the context of two nucleic acids or polypeptides refers to two or more sequences or subsequences that have at least about 60%, about 80%, about 90%, about 90-95%, about 95%, about 98%, about 99% or more nucleotide or amino acid residue identity, when compared and aligned for maximum correspondence using a sequence comparison algorithm or by visual inspection. Such "substantially identical" sequences are typically considered to be "homologous," without reference to actual ancestry. Preferably, the "substantial identity" between nucleotides exists over a region of the polynucleotide at least about 50 nucleotides in length, at least about 100 nucleotides in length, at least about 200 nucleotides in length, at least about 300 nucleotides in length, or at least about 500 nucleotides in length, most preferably over their entire length of the polynucleotide. Preferably, the "substantial identity" between polypeptides exists over a region of the polypeptide at least about 50 amino acid residues in length, more preferably over a region of at least about 100 amino acid residues, and most preferably, the sequences are substantially identical over their entire length.

The phrase "sequence similarity," in the context of two polypeptides refers to the extent of relatedness between two or more sequences or subsequences. Such sequences will typically have some degree of amino acid sequence identity, and in addition, where there exists amino acid non-identity, there is some percentage of substitutions within groups of functionally related amino acids. For example, substitution (misalignment) of a serine with a threonine in a polypeptide is sequence similarity (but not identity).

As used herein, the term "homologous" refers to two or more amino acid sequences when they are derived, naturally or artificially, from a common ancestral protein or amino acid sequence. Similarly, nucleotide sequences are homologous when they are derived, naturally or artificially, from a common ancestral nucleic acid. Homology in proteins is generally inferred from amino acid sequence identity and sequence similarity between two or more proteins. The precise percentage of identity and/or similarity between sequences that is useful in establishing homology varies with the nucleic acid and protein at issue, but as little as 25% sequence similarity is routinely used to establish homology. Higher levels of sequence similarity, *e.g.,* 30%, 40%, 50%, 60%, 70%, 80%, 90%, 95%, or 99% or more, can also be used to establish homology. Methods for determining sequence similarity percentages (*e.g.,* BLASTP and BLASTN using default parameters) are generally available.

As used herein, the terms "portion," "subsequence," "segment" or "fragment" or similar terms refer to any portion of a larger sequence (*e.g.,* a nucleotide subsequence or an amino acid subsequence) that is smaller than the complete sequence from which it was derived. The minimum length of a subsequence is generally not limited, except that a minimum length may be useful in view of its intended function. The subsequence can be derived from any portion of the parent molecule. In some aspects, the portion or subsequence retains a critical feature or biological activity of the larger molecule, or corresponds to a particular functional domain of the parent molecule, for example, the DNA-binding domain, or the transcriptional activation domain. Portions of polynucleotides can be any length, for example, at least 5, 10, 15, 20, 25, 30, 40, 50, 75, 100, 150, 200, 300 or 500 or more nucleotides in length.

As used herein, the term "kit" is used in reference to a combination of articles that facilitate a process, method, assay, analysis or manipulation of a sample. Kits can contain written instructions describing how to use the kit (e.g., instructions describing the methods of the present invention), chemical reagents or enzymes required for the method, primers and probes, as well as any other components.

### EXAMPLES

### EXAMPLE 1: DUAL-FLUORESCENT REPORTER PLASMID DESIGN AND CLONING

The reporter plasmid (pREP) was designed **(****Figure 1A****)** to have a backbone free of any potential methylation sites (CpG sequences) to ensure studied methylation effects are due to methylation of promoter regions only. The CpG free backbone consists of portions of a commercially available plasmid (pCpGfree VitroNMCS, Invivogen) that was heavily modified and supplemented with new sequence elements. The pCpGfree Vitro NMCS plasmid had several regions prone to homologous recombination removed and the bacterial I-EC2K promoter and Neo sequence (responsible for Kanamycin resistance in bacterial cells) was moved to a proximal location to the R6K origin of replication using standard molecular cloning techniques. mCherry (Nature Biotechnology 22, 1567 - 1572 (2004)) and mTAGBFP2 (PLoS One. 2011;6(12)) fluorescent proteins nucleotide sequences were modified from originally published sequences to remove all potential CpG methylation sites within the protein coding sequence. New sequences were synthesized as gblocks from Integrated DNA Technologies. The fluorescent protein sequences were inserted into the plasmids on opposing strands with Poly A transcription termination signal sequences downstream of both coding sequences. Any target promoter can be inserted upstream of the mCherry and an appropriate control promoter is inserted upstream of the mTAGBFP2 sequence for normalization purposes or to monitor off target effects.

Preliminary experiments show that a test promoter containing a CpG island shows over a 90% decrease in mCherry expression when fully methylated *in vitro* with a CpG MTase in comparison to an unmethylated plasmid (See **Figure 1B**). Both methylated and unmethylated plasmids show similar levels of BFP expression. Additionally, plasmids maintain the original methylation status even after being in cells for 48 hours.

### EXAMPLE 2: METHYLATED PROMOTER ASSAY PROTOCOL

### pREP plasmidpreparation: Full methylation of promoter region

The suspected methylation-sensitive target promoter is cloned upstream of the mCherry fluorescent protein. The control promoter is a CpG free EFla promoter cloned from the original CpGfree vitroNmcs plasmid (Invivogen) and is not sensitive to methylation. The pREP plasmid is methylated in vitro by the CpG methyltransferase, M.SssI (New England Biolabs®). In short, 30 µg of plasmid is mixed with 1 X NEB buffer 2, 600 µM SAM substrate and 16 U M.SssI enzyme. The mixture is incubated at 30°C overnight (12+ hours) and DNA purified using the QIAQuick® PCR Purification Kit (Qiagen®).

### In vivo gene expression assay

Plasmids can be transformed into any cell line for analysis. Currently all experiments have been done using the HEK293T cell line but cell lines can be changed if promoters have specific requirements. Cells are seeded at 0.75 x 10⁵ cells per well and allowed to grow overnight to approximately 50% confluence before transfection. Plasmids were transfected using Lipofectamine® 2000 (Invitrogen™) using manufacturer's recommendations. Transfection reagent and media is removed after 24 hours and replaced with fresh media. Cells are recovered at -48 hours after transfection for flow cytometry analysis. Recovered cells are spun in centrifuge at 400 g's for 10 minutes. Cells are washed once with 1 ml PBS and centrifuged again. PBS wash is removed and cells are resuspended in 500 µl PBS with 0.1% FBS. Cells are then filtered through a 70 micron filter and analyzed on a BD LSRFortessa™. Promoter strength is measured by the median fluorescence intensity of mCherry (measured at 610 nm after excitation using 561 nm laser) and normalized to mTAGBFP2 median fluorescent intensity (measured at 450 nm after excitation using 407 nm laser).

The pREP plasmid will contain both a target promoter controlling mCherry and a methylation-sensitive promoter controlling mTAGBFP2 expression to monitor off-target effects. By cotransfecting EME fusion constructs with the reporter plasmid we will rapidly select for specific modifications of the target promoter but not the control promoter. This reporter assay also allows for high-throughput FACS analysis of gene expression levels in addition to sorting of cells with desired phenotypes. pREP plasmids can be recovered for bisulfite sequencing analysis to determine specific methylation patterns.

### EXAMPLE 3: IN VIVO CAS9 REPRESSION OR ACTIVATION ASSAY

Experiments were performed using procedures similar to the previously described methylation assays. All experiments have been done using the HEK293T cell line but assays can be done in other cell lines depending on the user's needs. Cells are seeded at 0.75 x 10⁵ cells per well and allowed to grow overnight and should be ∼50% confluent before transfection. Plasmids were co-transfected using Lipofectamine® 2000 (Invitrogen™) using a ratio of 50 ng Cas9 expression plasmid: 450 ng pREP plasmid. Transfection reagent is incubated with cells 24 hours before media is removed and replaced. Cells are recovered at -48 hours for flow cytometry analysis using the BD LSRFortessa™ flow cytometer. Only cells expressing GFP (measured at 530 nm after excitation by 488 nm laser) and therefore also expressing dCas9 constructs were evaluated for mCherry and mTAGBFP2 median fluorescence intensity. **(****Figure 2****)**

### EXAMPLE 4: DUAL-FLUORESCENT REPORTER FOR PROMOTER TARGETING OF DCAS9 TRANSCRIPTIONAL ACTIVATORS OR REPRESSORS FOR TRANSIENT GENE EXPRESSION OR SILENCING

Localization of a deactivated Cas9 alone to certain regions of promoters can transiently repress activity by blocking transcription factors. In addition, transcriptional activators such as VP64 can be fused to Cas9 and localized in promoter regions to activate methylated or otherwise repressed genes. In many cases multiple Cas9 activators or repressors are required for optimal gene expression control. The dual-fluorescent reporter allows us to evaluate different and/or multiple Cas9 targeting guide strands to achieve desired levels of repression or activation. This could be an alternate strategy for regulating gene expression if short term expression changes are sufficient for therapeutic purposes or for short term research studies.

Stable cell lines will express either a deactivated Cas9 or dCas9-VP64 fusion to activate reporter plasmids. Screening will follow the general schemes described in **Figure 2** above. As a proof of concept transient transfection of dCas9 has been shown to reduce mCherry expression on a target promoter after cotransfection of the pREP plasmid and a dCas9 expression plasmid along with sgRNA's at different sites on the promoter **(****Figure 3****)**.

### EXAMPLE 5: GENERATING STABLE, INDUCIBLE CELL LINES

The tetracyclin-inducible system places expression of a gene-of-interest (GOI) under control of a Tet-Operator (Tet-O) promoter sequence. The sequence generally consists of seven repeats of a 19-base pair sequence separated by spacers, upstream of a minimal promoter (*e.g.,* CMV), with variations of this sequence designed to constrain "leakiness" of the promoter. Collectively this is referred to as the Tetracycline response element (TRE). Transcription from the TRE is activated by binding of the reverse tetracycline-controlled transactivator (rtTA) protein, which is expressed from a cell-specific promoter as a mutated tetracycline repressor sequence (TetR) fused to a transcription activator protein (VP16). In its native form rtTA does not bind the TRE; upon the addition and binding of tetracycline or its more stable analogue, doxycycline, to the rtTA, the complex is able to bind the TRE and transactivate expression of the GOI.

A mammalian cell line that stably and inducibly expresses the dCas9-split EME (SEME) proteins will be created. Our strategy requires generation of two stable integrants: a reverse tetracycline transactivator (rtTA), and the inducible dCas9-SEME fusion proteins. We will first generate a population of cells stably expressing the rtTA by transfecting a construct expressing the rtTA from an EF1α promoter, along with a fluorescent protein marker, into a mammalian cell line and selecting colonies by G418 selection. The resultant population will be FACS Turbo sorted on marker expression. The single cell clones will be expanded and screened for inducibility by transient transfection of a control plasmid expressing luciferase from the CMV-TetO promoter, with subsequent tetracycline treatment. This strategy will allow us to select a cell line that demonstrates high levels of induction and low levels of basal expression from the inducible promoter.

The cell lines demonstrating optimal characteristics will then be stably transfected with a vector expressing the dCas9-SEME fusion proteins under the control of a bidirectional CMV-TetO promoter. The bidirectional promoter allows for inducible, equimolar expression of both fusion partners simultaneously. This vector also constitutively expresses GFP, which will be monitored as a proxy for expression of the dCas9-SEME fragments by FACS Turbo sorting. GFP-positive cells will be expanded and characterized for speed of protein induction, levels of steady-state protein, and half-life of each fusion partner, by RT-qPCR in the presence of tetracycline. To measure the levels of dCas9-SEME proteins directly, we will perform Western blot using antibodies specific to the Cas9 proteins.

Equimolar expression of the SEME fragments may prove to be not ideal for reasons of toxicity and inefficient assembly of the fusion partners. If this is the case, we will clone the fusion proteins into a vector that expresses both proteins under the control of a single uni-directional inducible promoter. Inducible expression of both proteins would be made possible by inserting an IRES or a T2A/ E2A element between the fusion partners, resulting in concurrent expression of both proteins from the same mRNA, and naturally reduced expression of the downstream fusion partner. Furthermore, if the half-lives determined for the fusion proteins are too long, we will clone a PEST domain onto the termini of the SEME fragments, which will act as a signal peptide for protein degradation, substantially reducing protein half-life. Different PEST domains can be chosen that shorten protein half-life down to one hour.

### OTHER EMBODIMENTS

While the invention has been described in conjunction with the detailed description thereof, the foregoing description is intended to illustrate and not limit the scope of the invention, which is defined by the scope of the appended claims. Other aspects, advantages, and modifications are within the scope of the following claims.

## Claims

1. A mammalian cell expressing an expression cassette, wherein the expression cassette comprises:
a. a first promoter sequence operably linked to a nucleic acid sequence encoding (i) a fusion protein comprising a deactivated Cas9 and an epigenetic modifying enzyme and (ii) a selectable marker; and
b. a second promoter sequence operably linked to a nucleic acid sequence encoding at least one deactivated Cas9 specific trans-activating crRNA (TracrRNA),
wherein said cell is transfected with a reporter plasmid comprising a backbone that is free of any methylation sites, the reporter plasmid having a promoter sequence from a gene of interest inserted upstream of a nucleic acid encoding a first fluorescent protein and a control promoter sequences inserted upstream of a nucleic acid encoding a second fluorescent protein.

2. The mammalian cell expressing an expression cassette of claim 1, wherein the expression cassette further comprises a translation initiation sequence or self-cleaving peptide sequence located between the fusion protein and the selectable marker.

3. A mammalian cell expressing an expression cassette, wherein the expression cassette comprises:
a. a first promoter sequence operably linked to a nucleic acid sequence encoding (i) a first fusion protein comprising a deactivated Cas9 and a first portion of a bifurcated epigenetic modifying enzyme, (ii) and a second fusion protein comprising a deactivated Cas9 and a second portion of a bifurcated epigenetic modifying enzyme, and (iii) a selectable marker; and
b. a second promoter sequence operably linked to a nucleic acid sequence encoding at least one deactivated Cas9-specific trans-activating crRNA (TracrRNA),
wherein said cell is transfected with a reporter plasmid comprising a backbone that is free of any methylation sites, the reporter plasmid having a promoter sequence from a gene of interest inserted upstream of a nucleic acid encoding a first fluorescent protein and a control promoter sequences inserted upstream of a nucleic acid encoding a second fluorescent protein.

4. The mammalian cell expressing an expression cassette of claim 3, wherein the expression cassette further comprises a first translation initiation sequence or a self-cleaving peptide sequence located between the first fusion protein and the second fusion protein and a second translation initiation sequence or a self-cleaving peptide sequence located between the second fusion protein and the selectable marker.

5. The mammalian cell expressing an expression cassette of claim 2 or 4, wherein:
a. the translation initiation sequence is an internal ribosome entry site (IRES) sequence;or
b. the self-cleaving peptide sequence is a T2A sequence or an E2A sequence.

6. The mammalian cell expressing an expression cassette according to any one of the preceding claims, wherein the first promoter is a constitutive promoter or an inducible promoter.

7. A mammalian cell expressing an expression cassette, wherein the expression cassette comprises:
a. a first promoter sequence operably linked to a nucleic acid sequence encoding a first fusion protein comprising a deactivated Cas9 and a first portion of a bifurcated epigenetic modifying enzyme, and a second fusion protein comprising a deactivated Cas9 and a second portion of a bifurcated epigenetic modifying enzyme, wherein said first promoter sequence is a bidirectional inducible promoter;
b. a second promoter sequence operably linked to a selectable marker; and
c. a third promoter sequence operably linked to a nucleic acid sequence encoding at least one deactivated Cas9-specific trans-activating crRNA (TracrRNA),
wherein said cell is transfected with a reporter plasmid comprising a backbone that is free of any methylation sites, the reporter plasmid having a promoter sequence from a gene of interest inserted upstream of a nucleic acid encoding a first fluorescent protein and a control promoter sequences inserted upstream of a nucleic acid encoding a second fluorescent protein.

8. The mammalian cell expressing an expression cassette according to any one of the preceding claims, wherein the epigenetic modifying enzyme is a methyltransferase, a demethylase, or VP64.

9. The mammalian cell expressing an expression cassette of any one of the preceding claims, wherein the promoter from the gene of interest is methylation sensitive and the control promoter sequence is methylation insensitive.

10. The mammalian cell expressing an expression cassette of any one the preceding claims, wherein the promoter from the gene of interest and the control promoter sequence are methylation sensitive.

11. The mammalian cell expressing an expression cassette of claim 9, wherein the epigenetic modifying enzyme is a methyltransferase.

12. The mammalian cell expressing an expression cassette of claim 9, wherein the epigenetic modifying enzyme is a demethylase.

13. The mammalian cell expressing an expression cassette of claim 10, wherein the epigenetic modifying enzyme is a methyltransferase.

14. The mammalian cell expressing an expression cassette of claim 10, wherein the epigenetic modifying enzyme is a demethylase.

15. The mammalian cell expressing an expression cassette of any one of the preceding claims, wherein said cell is transfected with a plurality of crRNAs specific for the gene of interest.

16. A system comprising:
a. the mammalian cell expressing an expression cassette of any one of the preceding claims; and
b. a plurality of guide RNAs (gRNAs) specific for the gene of interest.

17. A method of determining the functionality of a dCAS9-epigenetic modifying enzyme fusion comprising:
a. contacting the mammalian cell expressing an expression cassette of any one of the preceding claims with a plurality of crRNAs specific for the gene of interest; and
b. detecting fluorescence of the first and second fluorescent protein, if present, wherein
i. the presence of fluorescence of the second fluorescent protein and the absence of fluorescence of the first fluorescent protein indicates that the dCAS9-fusion is functional; or
ii. the presence of fluorescence of the both the first and second indicates that the dCAS9-fusion is functional.

18. A method of identifying a functionally repressive CpG site in promoter of a gene of interest comprising:
a. contacting the mammalian cell expressing an expression cassette of claim 11 with a plurality of crRNAs specific for the gene of interest;
b. detecting fluorescence of the first and second fluorescent protein, if present;
c. identifying a cell expressing the second fluorescent protein and not the first fluorescent protein; and
d. performing bisulfite sequencing analysis on the cell of step (c) to identifying the functionally repressive CpG site.

19. A method of identifying a functionally repressive CpG site in promoter of a gene of interest comprising:
a. methylating the promoters in the reporter plasmid which is transfected into the mammalian cell expressing an expression cassette of claim 12;
b. contacting the cell of step (a) with a plurality of crRNAs specific for the gene of interest;
c. detecting fluorescence of the first and second fluorescent protein, if present;
d. identifying a cell expressing both the first fluorescent protein and the second fluorescent protein; and
e. performing bisulfite sequencing analysis on the cell of step (d) to identifying the functionally repressive CpG site.

20. A method of identifying a crRNA that specifically targets a promoter of a gene of interest comprising:
a. contacting the mammalian cell expressing an expression cassette of claim 13 with a plurality of crRNAs specific for the gene of interest;
b. detecting fluorescence of the first and second fluorescent protein, if present; and
c. identifying a cell expressing the second fluorescent protein and not the first fluorescent protein.

21. A method of identifying a crRNA that specifically targets a promoter of a gene of interest comprising:
a. methylating the promoters in the reporter plasmid which is transfected into the mammalian cell expressing an expression cassette of claim 14;
b. contacting the cell of step (a) with a plurality of crRNAs specific for the gene of interest;
c. detecting fluorescence of the first and second fluorescent protein, if present; and
d. identifying a cell expressing the second fluorescent protein and the not the first fluorescent protein.

## Patentansprüche

1. Säugetierzelle, die eine Expressionskassette exprimiert, wobei die Expressionskassette Folgendes umfasst:
a. eine erste Promotorsequenz, die funktionsfähig mit einer Nukleinsäuresequenz verknüpft ist, die (i) ein Fusionsprotein umfassend eine deaktivierte Cas9 und ein epigenetisch modifizierendes Enzym und (ii) einen selektierbaren Marker codiert;
b. eine zweite Promotorsequenz, die funktionsfähig mit einer Nukleinsäuresequenz verknüpft ist, die mindestens eine für eine deaktivierte Cas9 spezifische transaktivierende crRNA (TracrRNA) codiert,
wobei die Zelle mit einem Reporterplasmid transfiziert ist, das ein Grundgerüst umfasst, welches frei von jeglichen Methylierungsstellen ist, wobei das Reporterplasmid eine Promotorsequenz aus einem interessierenden Gen aufweist, die vorgeordnet zu einer Nukleinsäure eingefügt ist, welche ein erstes fluoreszierendes Protein codiert, und eine Kontrollpromotorsequenz aufweist, die vorgeordnet zu einer Nukleinsäure eingefügt ist, welche ein zweites fluoreszierendes Protein codiert.

2. Säugetierzelle, die eine Expressionskassette exprimiert, nach Anspruch 1, wobei die Expressionskassette ferner eine Translationsinitiationssequenz oder eine selbstspaltende Peptidsequenz umfasst, die sich zwischen dem Fusionsprotein und dem selektierbaren Marker befindet.

3. Säugetierzelle, die eine Expressionskassette exprimiert, wobei die Expressionskassette Folgendes umfasst:
a. eine erste Promotorsequenz, die funktionsfähig mit einer Nukleinsäuresequenz verknüpft ist, die (i) ein erstes Fusionsprotein umfassend eine deaktivierte Cas9 und einen ersten Abschnitt eines gegabelten, epigenetischen, modifizierenden Enzyms und (ii) ein zweites Fusionsprotein umfassend eine deaktivierte Cas9 und einen zweiten Abschnitt eines gegabelten, epigenetischen, modifizierenden Enzyms und (iii) einen selektierbaren Marker codiert; und
b. eine zweite Promotorsequenz, die funktionsfähig mit einer Nukleinsäuresequenz verknüpft ist, die mindestens eine für eine deaktivierte Cas9 spezifische transaktivierende crRNA (TracrRNA) codiert,
wobei die Zelle mit einem Reporterplasmid transfiziert ist, das ein Grundgerüst umfasst, welches frei von jeglichen Methylierungsstellen ist, wobei das Reporterplasmid eine Promotorsequenz aus einem interessierenden Gen aufweist, die vorgeordnet zu einer Nukleinsäure eingefügt ist, welche ein erstes fluoreszierendes Protein codiert, und eine Kontrollpromotorsequenz aufweist, die vorgeordnet zu einer Nukleinsäure eingefügt ist, welche ein zweites fluoreszierendes Protein codiert.

4. Säugetierzelle, die eine Expressionskassette exprimiert, nach Anspruch 3, wobei die Expressionskassette ferner eine erste Translationsinitiationssequenz oder eine selbstspaltende Peptidsequenz, die zwischen dem ersten Fusionsprotein und dem zweiten Fusionsprotein angeordnet ist, und eine zweite Translationsinitiationssequenz oder eine selbstspaltende Peptidsequenz, die zwischen dem zweiten Fusionsprotein und dem selektierbaren Marker angeordnet ist, umfasst.

5. Säugetierzelle, die eine Expressionskassette exprimiert, nach Anspruch 2 oder 4, wobei:
a. die Translationsinitiationssequenz eine Sequenz einer internen ribosomalen Eintrittsstelle (IRES) ist; oder
b. die selbstspaltende Peptidsequenz eine T2A-Sequenz oder eine E2A-Sequenz ist.

6. Säugetierzelle, die eine Expressionskassette exprimiert, nach einem der vorhergehenden Ansprüche, wobei der erste Promotor ein konstitutiver Promotor oder ein induzierbarer Promotor ist.

7. Säugetierzelle, die eine Expressionskassette exprimiert, wobei die Expressionskassette Folgendes umfasst:
a. eine erste Promotorsequenz, die funktionsfähig mit einer Nukleinsäuresequenz verknüpft ist, die ein erstes Fusionsprotein umfassend eine deaktivierte Cas9 und einen ersten Abschnitt eines gegabelten, epigenetischen, modifizierenden Enzyms und ein zweites Fusionsprotein umfassend eine deaktivierte Cas9 und einen zweiten Abschnitt eines gegabelten, epigenetischen, modifizierenden Enzyms umfasst, wobei die erste Promotorsequenz ein bidirektionaler induzierbarer Promotor ist;
b. eine zweite Promotorsequenz, die funktionsfähig mit einem selektierbaren Marker verknüpft ist; und
c. eine dritte Promotorsequenz, die funktionsfähig mit einer Nukleinsäuresequenz verknüpft ist, die mindestens eine für eine deaktivierte Cas9 spezifische transaktivierende crRNA (TracrRNA) codiert,
wobei die Zelle mit einem Reporterplasmid transfiziert ist, das ein Grundgerüst umfasst, welches frei von jeglichen Methylierungsstellen ist, wobei das Reporterplasmid eine Promotorsequenz aus einem interessierenden Gen aufweist, die vorgeordnet zu einer Nukleinsäure eingefügt ist, welche ein erstes fluoreszierendes Protein codiert, und eine Kontrollpromotorsequenz aufweist, die vorgeordnet zu einer Nukleinsäure eingefügt ist, welche ein zweites fluoreszierendes Protein codiert.

8. Säugetierzelle, die eine Expressionskassette exprimiert, nach einem der vorhergehenden Ansprüche, wobei das epigenetische modifizierende Enzym eine Methyltransferase, eine Demethylase oder VP64 ist.

9. Säugetierzelle, die eine Expressionskassette exprimiert, nach einem der vorhergehenden Ansprüche, wobei der Promotor aus dem interessierenden Gen methylierungsempfindlich und die Kontrollpromotorsequenz methylierungsunempfindlich ist.

10. Säugetierzelle, die eine Expressionskassette exprimiert, nach einem der vorhergehenden Ansprüche, wobei der Promotor aus dem interessierenden Gen und die Kontrollpromotorsequenz methylierungsempfindlich sind.

11. Säugetierzelle, die eine Expressionskassette exprimiert, nach Anspruch 9, wobei das epigenetische modifizierende Enzym eine Methyltransferase ist.

12. Säugetierzelle, die eine Expressionskassette exprimiert, nach Anspruch 9, wobei das epigenetische modifizierende Enzym eine Demethylase ist.

13. Säugetierzelle, die eine Expressionskassette exprimiert, nach Anspruch 10, wobei das epigenetische modifizierende Enzym eine Methyltransferase ist.

14. Säugetierzelle, die eine Expressionskassette exprimiert, nach Anspruch 10, wobei das epigenetische modifizierende Enzym eine Demethylase ist.

15. Säugetierzelle, die eine Expressionskassette exprimiert, nach einem der vorhergehenden Ansprüche, wobei die Zelle mit einer Vielzahl von crRNAs transfiziert ist, die für das interessierende Gen spezifisch sind.

16. System umfassend:
a. die Säugetierzelle, die eine Expressionskassette exprimiert, nach einem der vorhergehenden Ansprüche; und
b. eine Vielzahl von Leit-RNAs (guide RNAs, gRNAs), die für das interessierende Gen spezifisch sind.

17. Verfahren zum Bestimmen der Funktionalität einer dCAS9-epigenetischen modifizierenden Enzymfusion, umfassend:
a. Inkontaktbringen der Säugetierzelle, die eine Expressionskassette exprimiert, nach einem der vorhergehenden Ansprüche mit einer Vielzahl von crRNAs, die für das interessierende Gen spezifisch sind; und
b. Detektieren von Fluoreszenz des ersten und zweiten fluoreszierenden Proteins, falls vorhanden, wobei
i. das Vorhandensein von Fluoreszenz des zweiten fluoreszierenden Proteins und das Fehlen von Fluoreszenz des ersten fluoreszierenden Proteins anzeigt, dass die dCAS9-Fusion funktionsfähig ist; oder
ii. das Vorhandensein einer Fluoreszenz sowohl des ersten als auch des zweiten fluoreszierenden Proteins anzeigt, dass die dCAS9-Fusion funktionsfähig ist.

18. Verfahren zum Identifizieren einer funktionell repressiven CpG-Stelle im Promotor eines interessierenden Gens, umfassend:
a. Inkontaktbringen der Säugetierzelle, die eine Expressionskassette exprimiert, nach Anspruch 11 mit einer Vielzahl von crRNAs, die für das interessierende Gen spezifisch sind;
b. Detektieren von Fluoreszenz des ersten und zweiten fluoreszierenden Proteins, falls vorhanden;
c. Identifizieren einer Zelle, die das zweite fluoreszierende Protein und nicht das erste fluoreszierende Protein exprimiert; und
d. Durchführen einer Bisulfitsequenzierungsanalyse an der Zelle von Schritt (c) zum Identifizieren der funktionell repressiven CpG-Stelle.

19. Verfahren zum Identifizieren einer funktionell repressiven CpG-Stelle im Promotor eines interessierenden Gens, umfassend:
a. Methylieren der Promotoren in dem Reporterplasmid, das in die Säugetierzelle, die eine Expressionskassette exprimiert, nach Anspruch 12 transfiziert worden ist;
b. Inkontaktbringen der Zelle von Schritt (a) mit einer Vielzahl von crRNAs, die für das interessierende Gen spezifisch sind;
c. Detektieren von Fluoreszenz des ersten und zweiten fluoreszierenden Proteins, falls vorhanden;
d. Identifizieren einer Zelle, die sowohl das erste fluoreszierende Protein als auch das zweite fluoreszierende Protein exprimiert; und
e. Durchführen einer Bisulfitsequenzierungsanalyse an der Zelle von Schritt (d) zum Identifizieren der funktionell repressiven CpG-Stelle.

20. Verfahren zum Identifizieren einer crRNA, die spezifisch auf einen Promotor eines interessierenden Gens abzielt, umfassend:
a. Inkontaktbringen der Säugetierzelle, die eine Expressionskassette exprimiert, nach Anspruch 13 mit einer Vielzahl von crRNAs, die für das interessierende Gen spezifisch sind;
b. Detektieren von Fluoreszenz des ersten und zweiten fluoreszierenden Proteins, falls vorhanden; und
c. Identifizieren einer Zelle, die das zweite fluoreszierende Protein und nicht das erste fluoreszierende Protein exprimiert.

21. Verfahren zum Identifizieren einer crRNA, die spezifisch auf einen Promotor eines interessierenden Gens abzielt, umfassend:
a. Methylieren der Promotoren in dem Reporterplasmid, das in die Säugetierzelle, die eine Expressionskassette exprimiert, nach Anspruch 14 transfiziert worden ist;
b. Inkontaktbringen der Zelle von Schritt (a) mit einer Vielzahl von crRNAs, die für das interessierende Gen spezifisch sind;
c. Detektieren von Fluoreszenz des ersten und zweiten fluoreszierenden Proteins, falls vorhanden; und
d. Identifizieren einer Zelle, die das zweite fluoreszierende Protein und nicht das erste fluoreszierende Protein exprimiert.

## Revendications

1. Une cellule de mammifère exprimant une cassette d'expression, dans laquelle la cassette d'expression comprend :
a. une première séquence promotrice liée de manière fonctionnelle à une séquence d'acide nucléique codant pour (i) une protéine de fusion comprenant une Cas9 désactivée et une enzyme de modification épigénétique et (ii) un marqueur sélectionnable ; et
b. une deuxième séquence promotrice liée de manière fonctionnelle à une séquence d'acide nucléique codant pour au moins un ARNcr de transactivation spécifique de Cas9 désactivé (ARNtracr),
dans laquelle ladite cellule est transfectée avec un plasmide rapporteur comprenant une colonne vertébrale qui est dépourvue de tout site de méthylation, le plasmide rapporteur ayant une séquence promotrice issue d'un gène d'intérêt inséré en amont d'un acide nucléique codant pour une première protéine fluorescente et une séquence promotrice de contrôle insérée en amont d'un acide nucléique codant pour une deuxième protéine fluorescente.

2. La cellule de mammifère exprimant une cassette d'expression selon la revendication 1, dans laquelle la cassette d'expression comprend en outre une séquence de démarrage de la traduction ou une séquence peptidique à autoclivage située entre la protéine de fusion et le marqueur sélectionnable.

3. Une cellule de mammifère exprimant une cassette d'expression, dans laquelle la cassette d'expression comprend :
a. une première séquence promotrice liée de manière fonctionnelle à une séquence d'acide nucléique codant pour (i) une première protéine de fusion comprenant une Cas9 désactivée et une première partie d'une enzyme de modification épigénétique bifurquée, (ii) et une deuxième protéine de fusion comprenant une Cas9 désactivée et une deuxième partie d'une enzyme de modification épigénétique bifurquée, et (iii) un marqueur sélectionnable ; et
b. une deuxième séquence promotrice liée de manière fonctionnelle à une séquence d'acide nucléique codant pour au moins un ARNcr de transactivation spécifique de Cas9 désactivé (ARNtracr),
dans laquelle ladite cellule est transfectée avec un plasmide rapporteur comprenant une colonne vertébrale qui est dépourvue de tout site de méthylation, le plasmide rapporteur ayant une séquence promotrice issue d'un gène d'intérêt inséré en amont d'un acide nucléique codant pour une première protéine fluorescente et une séquence promotrice de contrôle insérée en amont d'un acide nucléique codant pour une deuxième protéine fluorescente.

4. La cellule de mammifère exprimant une cassette d'expression selon la revendication 3, dans laquelle la cassette d'expression comprend en outre une première séquence de démarrage de la traduction ou une séquence peptidique à autoclivage située entre la première protéine de fusion et la deuxième protéine de fusion et une deuxième séquence de démarrage de la traduction ou une séquence peptidique à autoclivage située entre la deuxième protéine de fusion et le marqueur sélectionnable.

5. La cellule de mammifère exprimant une cassette d'expression selon la revendication 2 ou 4, dans laquelle :
a. la séquence de démarrage de la traduction est une séquence du site d'entrée interne des ribosomes (IRES) ; ou
b. la séquence peptidique à autoclivage est une séquence T2A ou une séquence E2A.

6. La cellule de mammifère exprimant une cassette d'expression selon l'une quelconque des revendications précédentes, dans laquelle le premier promoteur est un promoteur constitutif ou un promoteur inductible.

7. Une cellule de mammifère exprimant une cassette d'expression, dans laquelle la cassette d'expression comprend :
a. une première séquence promotrice liée de manière fonctionnelle à une séquence d'acide nucléique codant pour une première protéine de fusion comprenant une Cas9 désactivée et une première partie d'une enzyme de modification épigénétique bifurquée, et une deuxième protéine de fusion comprenant une Cas9 désactivée et une deuxième partie d'une enzyme de modification épigénétique bifurquée, dans laquelle ladite première séquence promotrice est un promoteur inductible bidirectionnel ;
b. une deuxième séquence promotrice liée de manière fonctionnelle à un marqueur sélectionnable ; et
c. une troisième séquence promotrice liée de manière fonctionnelle à une séquence d'acide nucléique codant pour au moins un ARNcr de transactivation spécifique de Cas9 désactivé (ARNtracr),
dans laquelle ladite cellule est transfectée avec un plasmide rapporteur comprenant une colonne vertébrale qui est dépourvue de tout site de méthylation, le plasmide rapporteur ayant une séquence promotrice issue d'un gène d'intérêt inséré en amont d'un acide nucléique codant pour une première protéine fluorescente et une séquence promotrice de contrôle insérée en amont d'un acide nucléique codant pour une deuxième protéine fluorescente.

8. La cellule de mammifère exprimant une cassette d'expression selon l'une quelconque des revendications précédentes, dans laquelle l'enzyme de modification épigénétique est une méthyltransférase, une déméthylase, ou VP64.

9. La cellule de mammifère exprimant une cassette d'expression selon l'une quelconque des revendications précédentes, dans laquelle le promoteur issu du gène d'intérêt est sensible à la méthylation et la séquence promotrice de contrôle est insensible à la méthylation.

10. La cellule de mammifère exprimant une cassette d'expression selon l'une quelconque des revendications précédentes, dans laquelle le promoteur issu du gène d'intérêt et la séquence promotrice de contrôle sont sensibles à la méthylation.

11. La cellule de mammifère exprimant une cassette d'expression selon la revendication 9, dans laquelle l'enzyme de modification épigénétique est une méthyltransférase.

12. La cellule de mammifère exprimant une cassette d'expression selon la revendication 9, dans laquelle l'enzyme de modification épigénétique est une déméthylase.

13. La cellule de mammifère exprimant une cassette d'expression selon la revendication 10, dans laquelle l'enzyme de modification épigénétique est une méthyltransférase.

14. La cellule de mammifère exprimant une cassette d'expression selon la revendication 10, dans laquelle l'enzyme de modification épigénétique est une déméthylase.

15. La cellule de mammifère exprimant une cassette d'expression selon l'une quelconque des revendications précédentes, dans laquelle ladite cellule est transfectée avec une pluralité d'ARNcr spécifiques pour le gène d'intérêt.

16. Un système comprenant :
a. la cellule de mammifère exprimant une cassette d'expression selon l'une quelconque des revendications précédentes ; et
b. une pluralité d'ARN guides (ARNg) spécifiques pour le gène d'intérêt.

17. Un procédé de détermination de la fonctionnalité d'une fusion d'enzyme de modification épigénétique dCAS9 comprenant :
a. la mise en contact de la cellule de mammifère exprimant une cassette d'expression selon l'une quelconque des revendications précédentes avec une pluralité d'ARNcr spécifiques pour le gène d'intérêt ; et
b. la détection de la fluorescence des première et deuxième protéines fluorescentes, le cas échéant, dans laquelle
I. la présence de fluorescence de la deuxième protéine fluorescente et l'absence de fluorescence de la première protéine fluorescente indiquent que la fusion dCAS9 est fonctionnelle ; ou
II. la présence de fluorescence à la fois de la première et de la deuxième indique que la fusion dCAS9 est fonctionnelle.

18. Un procédé d'identification d'un site CpG fonctionnellement répressif dans le promoteur d'un gène d'intérêt comprenant :
a. la mise en contact de la cellule de mammifère exprimant une cassette d'expression selon la revendication 11 avec une pluralité d'ARNcr spécifiques pour le gène d'intérêt ;
b. la détection de la fluorescence des première et deuxième protéines fluorescentes, le cas échéant ;
c. l'identification d'une cellule exprimant la deuxième protéine fluorescente et non la première protéine fluorescente ; et
d. la réalisation d'une analyse de séquençage au bisulfite sur la cellule de l'étape (c) pour identifier le site CpG fonctionnellement répressif.

19. Un procédé d'identification d'un site CpG fonctionnellement répressif dans le promoteur d'un gène d'intérêt comprenant :
a. la méthylation des promoteurs dans le plasmide rapporteur qui est transfecté dans la cellule de mammifère exprimant une cassette d'expression selon la revendication 12 ;
b. la mise en contact de la cellule de l'étape (a) avec une pluralité d'ARNcr spécifiques pour le gène d'intérêt ;
c. la détection de la fluorescence des première et deuxième protéines fluorescentes, le cas échéant ;
d. l'identification d'une cellule exprimant à la fois la première protéine fluorescente et la deuxième protéine fluorescente ; et
e. la réalisation d'une analyse de séquençage au bisulfite sur la cellule de l'étape (d) pour identifier le site CpG fonctionnellement répressif.

20. Un procédé d'identification d'un ARNcr qui cible spécifiquement un promoteur d'un gène d'intérêt comprenant :
a. la mise en contact de la cellule de mammifère exprimant une cassette d'expression selon la revendication 13 avec une pluralité d'ARNcr spécifiques pour le gène d'intérêt ;
b. la détection de la fluorescence des première et deuxième protéines fluorescentes, le cas échéant ; et
c. l'identification d'une cellule exprimant la deuxième protéine fluorescente et non la première protéine fluorescente.

21. Un procédé d'identification d'un ARNcr qui cible spécifiquement un promoteur d'un gène d'intérêt comprenant :
a. la méthylation des promoteurs dans le plasmide rapporteur qui est transfecté dans la cellule de mammifère exprimant une cassette d'expression selon la revendication 14 ;
b. la mise en contact de la cellule de l'étape (a) avec une pluralité d'ARNcr spécifiques pour le gène d'intérêt ;
c. la détection de la fluorescence des première et deuxième protéines fluorescentes, le cas échéant ; et
d. l'identification d'une cellule exprimant la deuxième protéine fluorescente et non la première protéine fluorescente.
